# FASCICULE DE BREVET EUROPEEN

(11) **EP 2 144 565 B1**
(45) Date de publication et mention de la délivrance du brevet: **02.11.2011**
(21) Numéro de dépôt: 08805741.9
(22) Date de dépôt: 05.05.2008
(51) Int. Cl.: A61B 6/10, G21F 3/00

(54) **HOUSSE STERILE POUR PARAVENT EN MATERIAU RADIOPROTECTEUR**
STERILE ABDECKUNG FÜR EINEN SCHIRM AUS STRAHLENSCHÜTZENDEM MATERIAL
STERILE COVER FOR A SCREEN MADE OF RADIOPROTECTIVE MATERIAL

(30) Priorité: 09.05.2007 FR 0703316
(43) Date de publication de la demande: 20.01.2010
(73) Titulaire: LEMER PROTECTION ANTI-X PAR ABREVIATION SOCIETE LEMER PAX, 44470 Carquefou (FR)
(72) Inventeur: LEMER, Pierre-Marie, F-44000 Nantes (FR)
(74) Mandataire: Michelet, Alain
(86) Numéro de dépôt international: PCT/FR2008/050787
(87) Numéro de publication internationale: WO 2008/148991

(56) Documents cités:
- WO-A-03/088267
- WO-A-2004/107979

## Description

La présente invention concerne une housse stérile pour un paravent radioprotecteur adapté pour assurer la protection d'un opérateur contre les rayonnements ionisants de type rayons X ou autres.

Certains actes médicaux nécessitent l'emploi de produits injectables émettant des rayonnements ionisants (c'est notamment le cas des opérations de cathétérisme, de pose de pacemaker, mais aussi de certains examens vasculaires, neurologiques et urologiques), ou mettent en oeuvre directement des rayonnements ionisants pour réaliser des diagnostics ou des traitements, par exemple pour les techniques de fibrillation auriculaire en électrophysiologie.

Au cours de telles interventions, l'opérateur (technicien, médecin, chirurgien ou autre) doit être protégé contre les rayonnements auxquels le patient est soumis.

Pour cela, il peut porter des vêtements du genre blouse, chasuble ou tablier, réalisés en matériau radioprotecteur. Cependant, ces vêtements de protection ne permettent pas des conditions optimales de travail, en particulier du fait de leur poids important et de la forte transpiration qu'ils provoquent.

Pour se prémunir des dangers des rayonnements ionisants, une alternative intéressante consiste à mener les interventions médicales derrière un écran ou un paravent constitué de panneaux réalisés en matériau radioprotecteur.
Les conditions de travail, sans port de vêtements lourds, permettent alors d'intervenir avec une plus grande précision et une meilleure efficacité, ceci en respectant des conditions de sécurité toujours optimales.

Pour la plupart des interventions, un film plastique stérile, formant housse, est rapporté pour recouvrir les différentes faces du paravent, de façon à mener l'intervention dans des conditions sanitaires satisfaisantes ; cette housse stérile est remplacée à chaque intervention.
Ce type de housse stérile permet d'éviter une contamination infectieuse des patients, notamment par phénomène de transfert et de dispersion de germes bactériens ou de virus qui seraient présents sur le paravent employé lors de l'intervention.

Une structure de paravent radioprotecteur qui est particulièrement efficace et intéressante est décrite dans le document WO-03/088267.
Le paravent correspondant est constitué d'une paroi frontale associée à une paroi latérale s'étendant à l'équerre ou sensiblement à l'équerre à partir de l'un des côtés de ladite paroi frontale ; ces deux parois comportent des panneaux transparents sur une partie au moins de leur hauteur. La partie supérieure de la paroi frontale est inclinée vers l'avant, formant surplomb, pour permettre à l'opérateur de se rapprocher de la zone d'intervention ; cette paroi frontale est encore munie de deux orifices pour le passage des bras de l'opérateur : - un premier orifice de passage est aménagé à proximité de l'angle formé avec la paroi latérale, et - un second orifice de passage, ouvert latéralement, est situé sur son bord libre, présentant ainsi une forme générale d'encoche en arc de cercle ou en U orienté horizontalement.
Cette structure de paravent radioprotecteur procure, d'une part, une bonne visibilité pour l'opérateur, et d'autre part, un grand confort au niveau de son positionnement dans le cadre de l'intervention.

Or, les housses stériles actuelles ne sont pas adaptées pour les paravents de cette forme. En outre, elles se composent de plusieurs pièces devant être solidarisées ensemble lors de la mise en place sur le paravent, ce qui oblige la coopération de plusieurs personnes, et ce qui rend cette procédure peu simple et peu rapide. De plus, en fonction de la structure du paravent, son recouvrement n'est pas toujours efficace. Le document WO 2004/107979 décrit également une housse stérile pour un paravent radioprotecteur.

Pour ces raisons, la demanderesse a développé une nouvelle housse stérile pour paravent radioprotecteur selon la revendication 1 et du type décrit dans le document WO-03/088267, dont la structure permet un montage simple, rapide et efficace, cela éventuellement par une personne seule.
Une fois mis en place, cet équipement stérile est parfaitement maintenu sur le paravent, avec un risque limité de décrochement ; cette housse assure encore un recouvrement efficace des différentes parties à protéger (en particulier des orifices de passage de bras).

En l'occurrence, la housse stérile conforme à l'invention se compose d'une seule pièce, qui comporte deux panneaux, l'un dit « panneau intérieur » et l'autre dit « panneau extérieur », lesquels panneaux sont conformés pour venir recouvrir respectivement au moins les faces intérieure et extérieure de la paroi frontale du paravent, cela sur au moins une partie de leur hauteur s'étendant au-dessous et au-dessus des orifices de passage des bras associés. Ces panneaux intérieur et extérieur sont reliés par une zone de jonction conformée pour venir épouser au moins une partie de la hauteur du bord libre de la paroi frontale, ladite zone de jonction comportant une échancrure conformée pour venir épouser la bordure de l'orifice de passage ouvert latéralement ; et ces panneaux intérieur et extérieur sont chacun munis d'une ouverture destinée à venir se placer de part et d'autre de l'autre orifice de passage, l'ouverture de l'un desdits panneaux étant munie d'un élément tubulaire souple formant une sorte de manchette, ladite manchette étant destinée à être passée au travers dudit orifice en regard, cela de sorte à recouvrir sa bordure et garantir son asepsie.

Selon une caractéristique complémentaire, les panneaux intérieur et/ou extérieur sont avantageusement prolongés par un ou des panneaux destinés à venir recouvrir les faces intérieure et/ou extérieure de la paroi latérale.

Selon une autre caractéristique de l'invention, pour un paravent radioprotecteur dont le premier orifice de passage est prolongé vers l'avant par un manchon de protection réalisé en un matériau radioprotecteur, l'ouverture du panneau intérieur est prolongée par une manchette souple dont la longueur est apte à recouvrir - la surface interne dudit manchon de protection, et aussi - au moins une partie de la longueur de sa surface externe par repliement. De manière avantageuse, l'extrémité libre de la manchette du panneau intérieur est munie d'un organe élastique, ce dernier étant agencé pour tendre à réduire son diamètre et étant destiné à former une sorte de bracelet de serrage apte à se resserrer sur la surface extérieure du manchon de protection (après passage au travers dudit manchon, et repliement extérieur sur lui-même) ; en outre, cette manchette du panneau intérieur peut être recouverte d'un élément amovible en forme de gaine dont l'une des extrémités est fermée, ladite gaine étant destinée à faciliter l'opération de passage de ladite manchette au travers du manchon de protection et étant destinée à être retirée après ladite opération de passage.
Toujours dans ce cas, l'ouverture du panneau extérieur est avantageusement également prolongée par une manchette souple, cette dernière étant destinée à venir recouvrir au moins une partie de la longueur de la surface externe du manchon de protection.

D'autre part, de manière particulièrement intéressante, la housse stérile comporte - une bande supérieure réalisée en matériau plastique transparent, et - une partie réalisée en matériau non tissé absorbant de type chirurgical, correspondant à une bande inférieure de ladite housse et/ou à la ou aux manchettes équipant les panneaux intérieur et/ou extérieur, et/ou à la zone de jonction entre lesdits panneaux ; les bordures attenantes entre le matériau plastique transparent et ledit matériau absorbant sont reliées par couture(s).

En outre, cette housse comporte avantageusement des moyens de solidarisation avec le paravent ; de préférence, ces moyens de solidarisation consistent en des ventouses judicieusement réparties au niveau de la bordure supérieure de la housse. De manière complémentaire ou alternative, ces moyens de solidarisation peuvent aussi se présenter sous la forme de bandes de matière adhésive ménagées sur au moins une partie de la longueur de la bordure supérieure de la housse et/ou sur au moins une partie de la hauteur de l'une au moins de ses bordures d'extrémités.

L'invention sera encore illustrée, sans être aucunement limitée, par la description suivante d'un mode de réalisation particulier, donné uniquement à titre d'exemple et représenté sur les dessins annexés dans lesquels :
- la figure 1 est une vue générale et en perspective, orientée de son côté avant, d'un paravent radioprotecteur destiné à être recouvert partiellement par la housse stérile conforme à l'invention ;
- la figure 2 est une vue schématique d'une housse stérile selon l'invention, représentée approximativement développée et vue du côté de sa face extérieure, adaptée pour recouvrir le paravent de la figure 1 ;
- la figure 3 est une vue générale et en perspective de la housse de la figure 2, partiellement pliée autour de la zone de jonction entre le panneau extérieur et le panneau intérieur ;
- la figure 4 montre le paravent radioprotecteur de la figure 1, vu en perspective par l'avant, maintenant équipé de la housse stérile des figures 2 et 3 ;
- la figure 5 correspond au paravent de la figure 4, vu en perspective par l'arrière ;
- la figure 6 est une vue en coupe selon VI-VI de la figure 5, c'est-à-dire selon un plan horizontal passant par les orifices du paravent qui permettent le passage des bras de l'opérateur, de manière à montrer schématiquement l'agencement de la housse stérile en position.

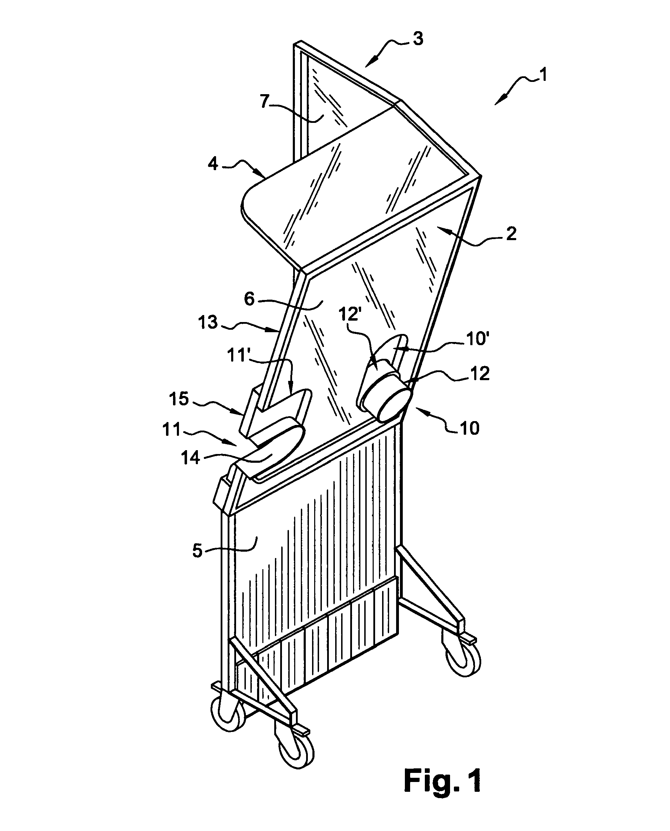

Le paravent radioprotecteur destiné à être recouvert par la housse stérile conforme à l'invention est décrit en détail dans le document WO-A-03/088267, et il correspond à l'objet de la figure 1.

Ce paravent radioprotecteur 1 comporte principalement une paroi frontale 2 reliée à une paroi latérale verticale 3, disposée à l'équerre ou sensiblement à l'équerre. Une paroi supplémentaire 4, faisant office de plafond, est ici avantageusement prévue entre les rebords supérieurs des parois frontale 2 et latérale 3 précitées.

Ce genre de paravent, destiné à protéger un opérateur contre les émissions de rayonnements ionisants, est constitué de plaques rigides en matériau radioprotecteur approprié.
Les différentes plaques peuvent être portées par une ossature métallique, par exemple en aluminium, qui intègre un blindage permettant une continuité de radioprotection au niveau de toutes les jonctions. La structure peut aussi, entièrement ou partiellement être réalisée par moulage de matériau radioprotecteur.

En l'occurrence, la paroi frontale 2 comprend une partie inférieure 5 constituée d'une plaque opaque verticale, et une partie supérieure 6 constituée par une plaque transparente.
Cette plaque transparente 6 est prévue inclinée vers l'avant, c'est-à-dire vers le champ opératoire. Son inclinaison permet à l'opérateur de se pencher vers l'avant au moment de son intervention, et ainsi de se rapprocher de la zone opératoire, notamment pour une meilleure visibilité et un plus grand confort.

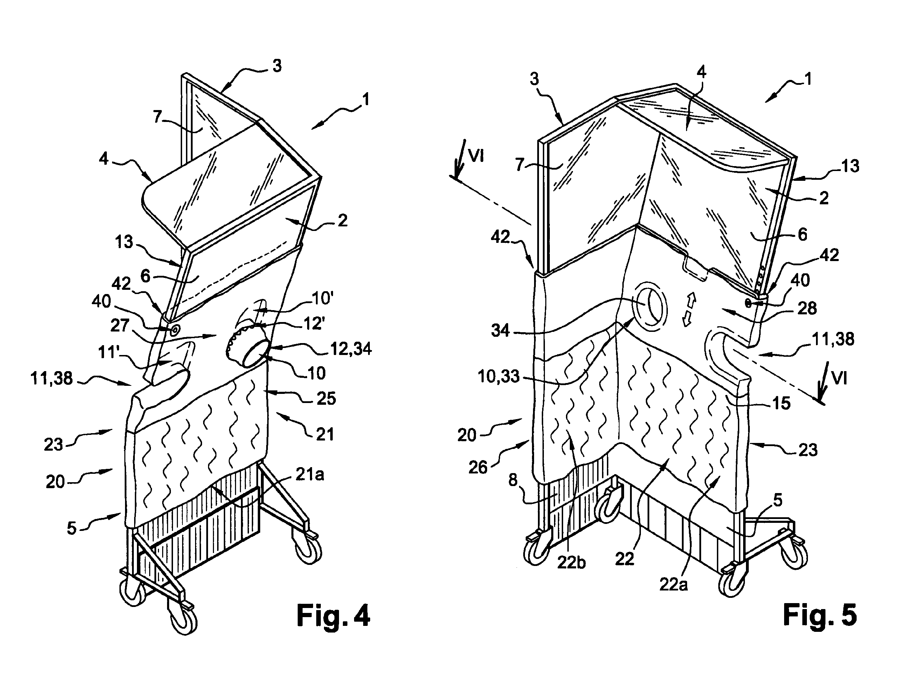

La paroi latérale 3 s'étend verticalement sur le côté. Elle comporte aussi une plaque supérieure transparente 7 et une plaque inférieure opaque 8 (visible seulement sur la figure 5). La plaque transparente 7 vise à procurer une visibilité latérale à l'opérateur, en particulier pour surveiller le patient ou accéder visuellement aux appareillages environnants.
Cette paroi latérale 3 épouse la forme en dièdre de la paroi frontale 2 ; le panneau transparent 7 a ainsi une forme générale sensiblement trapézoïdale.

Les dimensions générales du paravent sont adaptées pour permettre l'accueil d'opérateurs ayant des gabarits très différents.
Par exemple, la paroi frontale 2 et la paroi latérale 3 peuvent avoir une hauteur de l'ordre de 2 m.
Le panneau inférieur 5 de la paroi frontale 2 s'étend de préférence jusqu'à un niveau qui correspond à celui de la table d'opération ; à titre indicatif, ce panneau inférieur peut s'étendre sur une hauteur comprise entre 60 cm et 100 cm, de préférence voisine de 80 cm. Le panneau supérieur transparent 6 s'étend de préférence depuis le niveau de la table d'opération, c'est-à-dire entre environ 80 cm du sol et une hauteur de l'ordre de 2 m.

La plaque supérieure transparente 6 de la paroi frontale 1 est encore munie de deux orifices 10 et 11 de passage des bras de l'opérateur, pour permettre à ce dernier d'intervenir sur le patient.

L'un des orifices 10 (dit « premier orifice ») est de forme générale circulaire. Cet orifice 10 est situé du côté et à proximité de l'angle formé par les parois frontale 2 et latérale 3 ; il est adapté ici pour le passage du bras gauche de l'opérateur.
Pour augmenter la protection de l'opérateur, l'orifice 10 est prolongé par un manchon de protection 12 (par exemple en forme d'« iris ») monté sur un support tubulaire 12' adapté. Ce manchon de protection 12 s'étend en saillie vers l'avant, là où se trouvera le patient lors de l'intervention du praticien.

L'autre orifice 11 (dit « second orifice »), destiné ici à recevoir le bras droit de l'opérateur, est quant à lui ménagé au niveau de la bordure libre 13 de la paroi frontale 2, c'est-à-dire au niveau de la bordure opposée à la paroi latérale 3. Cet orifice 11 est ouvert latéralement, et se présente ainsi sous la forme générale d'une encoche en arc de cercle ou en U orientée horizontalement. Cet orifice 11 est ici avantageusement équipé d'une bague démontable 14.
L'orifice 11 en question permet à l'opérateur d'engager et de dégager facilement son bras, et ainsi de conserver une grande liberté de mouvement.

Des moyens structurels particuliers sont prévus pour permettre le déplacement vertical des orifices 10 et 11, sur la hauteur de la plaque transparente 6, de manière à pouvoir adapter leur niveau selon la taille de l'opérateur. Ainsi, ces orifices 10 et 11 sont ménagés dans la plaque transparente 6, et aussi dans un panneau de doublage 15 prévu à l'intérieur du paravent 1.
Ce panneau de doublage interne 15, qui comporte le manchon de protection 12 et la bague 14 précités, est prévu mobile parallèlement à ladite plaque transparente 6, guidé par des glissières latérales (non visibles) ; sa position est par exemple établie au moyen d'un index coopérant avec un doigt d'ancrage (non visibles).
La plaque transparente 6 comporte des ouvertures 10' et 11' de forme oblongue, surdimensionnées par rapport aux orifices 10 et 11 précités, et dont les dimensions, formes et positionnements sont adaptés à la course de la plaque de doublage 15 (et surtout du manchon 12 et de la bague 14 associés).

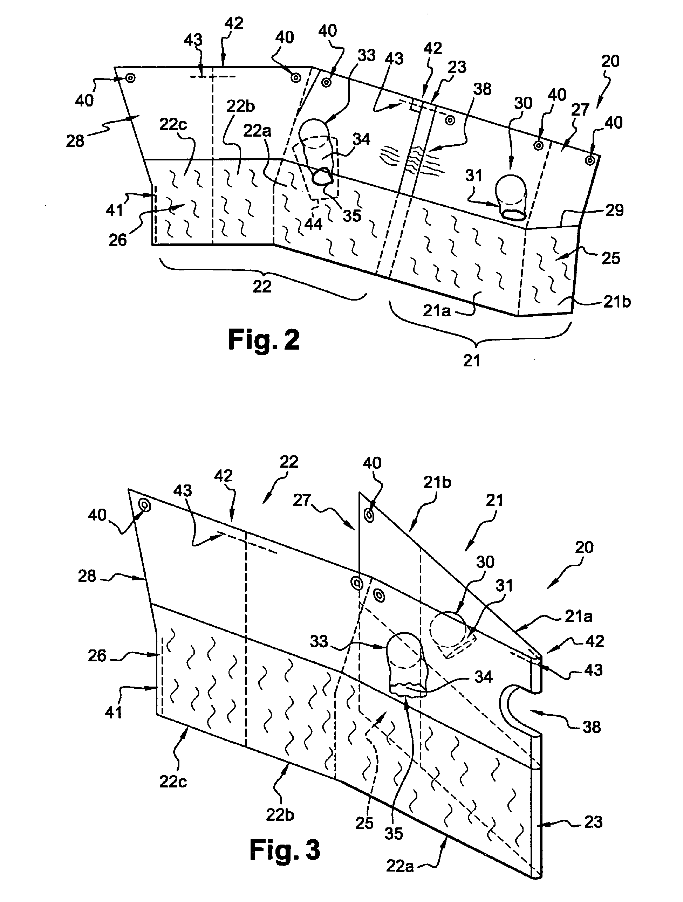

La housse stérile 20 conforme à l'invention, représentée à plat sur la figure 2, et partiellement mise en volume sur la figure 3, est adaptée pour recouvrir partiellement le paravent radioprotecteur 1 décrit ci-dessus en relation avec la figure 1.

Cette housse stérile 20, à plat (figure 2), est en forme de bande ou membrane longitudinale, composée principalement de deux parties 21 et 22, dont les bordures en regard sont reliées par une bande transversale 23 formant une zone de jonction transversale.
A titre indicatif, les dimensions des parties 21 et 22 de la housse 20 sont respectivement environ de 100 cm x 100 cm et 150 cm x 100 cm. De manière générale, elles sont adaptées aux dimensions du paravent radioprotecteur 1.

La housse 20 est en une seule pièce, réalisée par l'assemblage de deux matériaux souples adaptés à l'usage médical.
Plus précisément, les deux parties 21 et 22 comprennent chacune :
- une bande « inférieure » 25 ou 26, en matériau non tissé absorbant de type chirurgical, par exemple à base de polypropylène 40 g/m², et
- une bande « supérieure » transparente 27 ou 28, consistant en un film plastique transparent souple, par exemple de type polypropylène transparent d'une épaisseur de l'ordre de 70 µm.

La bande « inférieure » 25, 26 s'étend sur toute la longueur de la housse 20, tout comme la bande « supérieure » 27, 28 ; ces deux bandes longitudinales sont séparées par une ligne de jonction 29.

La zone de jonction 23 est quant à elle réalisée en matériau non tissé absorbant de type chirurgical, identique à celui employé pour les bandes inférieures 25 et 26 des panneaux 21 et 22.

Les bandes en matériau absorbant 23, 25, 26 et les bandes en matière plastique transparente 27 et 28 sont solidarisées ensemble, au niveau de leurs bordures en regard, par des moyens de liaison de type couture.

Plus précisément, la première partie 21 (située à droite sur la figure 2) se compose encore de deux panneaux 21a et 21b, destinés à venir recouvrir respectivement la face avant de la paroi frontale 2 du paravent et une partie de la face avant de sa paroi latérale 3.
Le panneau 21a en question, appelé par la suite « panneau extérieur » pour simplifier la description, a une largeur correspondant à celle de la paroi frontale 2 du paravent 1. Ce panneau extérieur 21a comporte, au niveau de la bande supérieure transparente 27, une ouverture circulaire 30 prolongée par une manchette 31, réalisée en matériau souple non tissé (du type de celui employé pour les bandes inférieures 25 et 26). L'autre panneau 21b de cette première partie 21 a une largeur correspondant à une partie de la largeur de la paroi latérale 3, et en l'occurrence un peu plus de la moitié de cette largeur.

La seconde partie 22 de la housse stérile 20 (située à gauche de la zone de jonction 23 sur la figure 2) se compose de trois panneaux 22a, 22b et 22c destinés à venir recouvrir, respectivement, la face intérieure de la paroi frontale 2 (ou autrement dit sa face arrière), la face intérieure de la paroi latérale 3 et une partie de la face extérieure de ladite paroi latérale 3.
Le panneau 22a a une largeur correspondant à celle de la paroi frontale 2. Une ouverture circulaire 33 est prévue dans sa bande transparente 28 ; cette ouverture 33 est ménagée de manière symétrique à l'ouverture 30 du panneau extérieur 21a, cela par rapport à la zone de jonction 23.
Là encore, cette ouverture 33 est prolongée par une manchette 34 ; la bordure libre de cette dernière est équipée d'un organe élastique 35 adapté pour tendre à en réduire son diamètre.
On note encore que cette manchette 34 présente une longueur supérieure à la manchette 31 du panneau extérieur 21a.

La zone de jonction 23 est destinée à venir épouser la bordure libre 13 de la paroi frontale 2. Cette zone de jonction 23 a ainsi une largeur correspondant environ à l'épaisseur de cette paroi frontale 2.
Comme on peut le voir en particulier sur la figure 3, cette zone 23 comporte un tronçon 38 formant une sorte d'échancrure, en forme générale de U orienté horizontalement, et disposé à un niveau situé entre les deux ouvertures de passage de bras 30 et 33 ménagées dans les panneaux 21a et 22a. Cette échancrure 38 a une forme et des dimensions adaptées pour venir épouser, au plus près, la bordure intérieure de l'orifice de passage de bras 11, en forme d'encoche débouchante, du paravent 1.

La housse 20 est encore munie de plusieurs ventouses 40, pour permettre son montage sur le paravent 1, ménagées sur la longueur de sa bordure supérieure, du côté orienté vers le paravent. De plus, une bande adhésive 41 est ménagée sur la partie inférieure de la bordure latérale libre du panneau 26, toujours du côté orienté vers le paravent.
L'adhésif utilisé est avantageusement du type « repositionnable ».

La forme de la ligne de jonction entre les panneaux 22a et 22b, et entre les panneaux 21a et 21b, est adaptée à l'inclinaison du panneau transparent 6 du paravent.
De plus, d'une manière générale, les formes et dimensions des différents panneaux constitutifs de la housse 20 sont prévus pour permettre l'adaptation de cette house 20 sur une gamme de paravents ayant de légères variations dimensionnelles. Pour optimiser cette adaptation, des zones de resserrement élastiques 42 sont ménagées au niveau des extrémités supérieures des bandes de la housse destinées à venir recouvrir les bordures latérales libres du paravent ; ces zones de resserrement 42 sont obtenues au moyen de bandes élastiques 43 cousues de manière appropriée sur les panneaux transparents 27 et 28, pour obtenir une traction de la matière dans le sens longitudinal.

Ainsi, la housse 20 consiste en une bande de matière destinée à envelopper une partie de la hauteur du paravent 1, de part et d'autre des orifices 10, 11 de passage des bras, c'est-à-dire au-dessus et au-dessous desdits orifices. La hauteur « d'enveloppement » du paravent peut être comprise entre 80 et 120 cm ; elle est avantageusement de l'ordre de 100 cm.
La partie supérieure de la housse 20 (constituée par les panneaux transparents 27 et 28) permet au praticien d'accéder visuellement au champ opératoire, du fait de sa transparence ; de préférence, cette partie supérieure 27, 28 ne s'étend pas trop en hauteur, de manière à ne pas perturber l'accès visuel aux matériels de contrôle situés en hauteur (le film plastique transparent constituant malgré tout un élément légèrement « perturbateur » de visibilité).
La partie inférieure en non tissé de la housse 20 (constituée par les panneaux 25 et 26) permet de rappeler la technicité du produit ; elle dispose d'une capacité d'absorption des liquides susceptible de trouver un intérêt dans la présente application.

Le montage de la housse 20 sur le paravent 1, s'effectue de manière très simple et rapide, comme détaillé ci-dessous en relation avec les figures 4 à 6.

A l'origine, la housse 20 est conditionnée stérilement dans un sachet hermétiquement fermé, dans lequel elle est convenablement pliée pour réduire son encombrement.
Au sein de ce conditionnement, la manchette 34 du panneau intérieur 22a est avantageusement logée dans une gaine 44 illustrée en traits pointillés sur la figure 2. Cette gaine 44, obturée à l'une de ses extrémités, est destinée à faciliter la pose « stérile » de la housse sur le paravent.
Pour le montage, l'opérateur doit tout d'abord sortir la housse de son sachet, puis la déployer ; il convient pour cela de prendre toutes les précautions habituelles pour ne pas contaminer le matériel (port de gants, d'une blouse, etc.).

L'opérateur positionne ensuite convenablement les différents panneaux 21a, 22a, 22b, 21b et 22c de la housse 20 contre les différences faces du paravent 1, ceci en positionnant la zone de jonction 23 en regard de la bordure libre 13 de la paroi frontale 2, et en faisant se recouvrir partiellement les bordures verticales libres desdits panneaux d'extrémités 21b et 22c contre la face externe de la paroi latérale 3.

Le positionnement convenable correspondant est réalisé en plaçant les orifices 30 et 33 de la housse en regard de l'orifice 10 du paravent, respectivement côtés extérieur et intérieur de la paroi frontale 2, et en utilisant les ventouses 40.

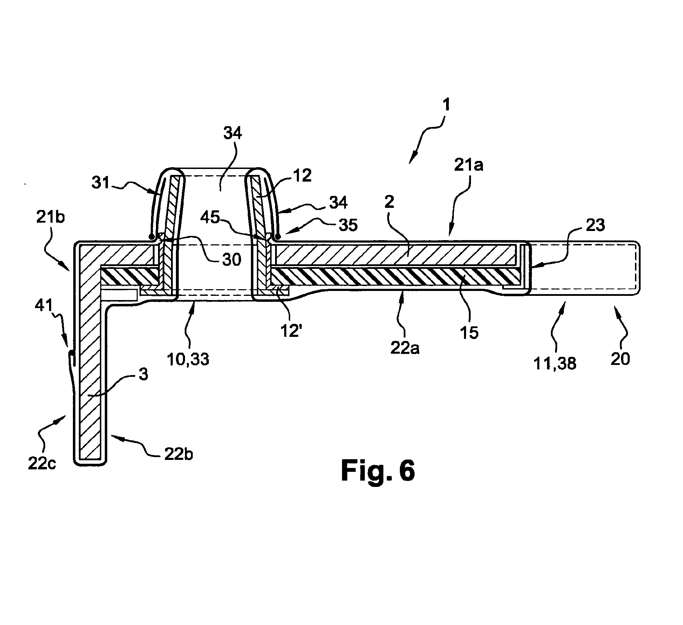

La manchette 31 de la housse vient alors recouvrir la surface externe du support tubulaire 12' et du manchon radioprotecteur 12 ; ensuite l'opérateur passe la manchette 34 de l'intérieur vers l'extérieur, au travers de l'orifice 10, cette opération étant facilitée par la gaine 44 précitée.
Une fois cette opération réalisée, l'opérateur retire la gaine 44 ; il recourbe l'extrémité libre élastique 35 de la manchette 34 autour du manchon 12, cela en direction de la paroi frontale 2, de sorte qu'elle recouvre également la surface extérieure périphérique dudit manchon de protection 12 et de sa manchette 31 associée (comme on peut le voir sur la figure 6) ; l'extrémité libre élastique 35 de la manchette 34 vient alors s'insérer dans une rainure annulaire 45 ménagée au niveau du support tubulaire 12' (voir encore figure 6).
On note que la manchette 34 est ainsi maintenue efficacement au travers de l'orifice de passage 10, et est apte en particulier à résister aux mouvements d'insertion et d'extraction du bras du praticien au travers de cet orifice 10.

Dans le même temps, l'opérateur doit veiller à ce que la zone de jonction 23 vienne épouser correctement la bordure libre 13 de la paroi frontale 2, et que son échancrure 38 vienne se loger dans l'orifice latéral de passage de bras 11.

D'une manière générale, en pratique, il s'avère intéressant:
- d'habiller tout d'abord la face externe de la paroi frontale 2 du paravent, en fixant la bordure supérieure du panneau 21a au moyen des ventouses associées 40,
- de positionner le panneau latéral 21b contre la face externe de la paroi latérale 3 en utilisant ses ventouses 40,
- de terminer l'habillage par le placage successif des panneaux 22a, 22b et 22c, en utilisant leurs propres ventouses 40.

La bande adhésive 41 est utilisée au final pour solidariser la partie inférieure du panneau 22c avec la face extérieure du panneau 21b.

Les ventouses 40 étant repositionnables, l'opérateur peut ajuster la position de la housse 20 de manière à ce qu'elle épouse au mieux le paravent.

Une fois la housse 20 convenablement mise en place (comme représenté sur les figures 4 à 6) le praticien peut mettre en oeuvre l'intervention médicale envisagée sur le patient.
On note que les bandes transparentes 27 et 28 sont agencées en regard des panneaux transparents 6 et 7 du paravent 1 (cela sur une partie de leur hauteur), ce qui permet de conserver un confort visuel efficace tout en assurant des conditions optimales d'hygiène pour le patient. De même, les orifices de passages de bras 10 et 11 sont protégés respectivement par la manchette stérile 34 et par l'échancrure 38, réduisant encore le risque infectieux pour le patient.

Ce type de housse stérile est à usage unique et lorsque l'intervention médicale est terminée, il suffit d'exercer de simples tractions pour la séparer du paravent.

De manière générale, on comprend bien que le montage de cette housse stérile est particulièrement simple et rapide et peut être mis en oeuvre par un opérateur unique. Une telle housse présente en plus l'intérêt d'assurer un recouvrement optimal des zones du paravent radioprotecteur susceptibles d'être en contact avec les mains ou les bras du praticien.

## Revendications

1. Housse stérile destinée à recouvrir au moins une partie de la hauteur d'un paravent (1) en matériau radioprotecteur assurant la protection d'un opérateur contre les émissions de rayonnements ionisants de type rayons X ou autres, lequel paravent (1), du type à section horizontale en L, est constitué d'une paroi frontale (2) associée à une paroi latérale (3) s'étendant à l'équerre ou sensiblement à l'équerre à partir de ladite paroi frontale (2), laquelle paroi frontale (2) est munie de deux orifices (10, 11) pour le passage des bras dudit opérateur, un premier orifice de passage (10) étant ménagé à proximité de l'angle formé par lesdites parois frontale (2) et latérale (3), un second orifice de passage (11) étant situé sur le bord libre (13) de ladite paroi frontale (2) et étant ouvert latéralement, présentant ainsi une forme générale d'encoche en arc de cercle ou de U orienté horizontalement,
**caractérisée en ce que** ladite housse stérile (20) se présente sous la forme d'une seule pièce, qui comporte au moins deux panneaux, l'un dit « panneau intérieur » (22a) et l'autre dit « panneau extérieur » (21a), conformés pour venir recouvrir respectivement au moins les faces intérieure et extérieure de ladite paroi frontale (2), cela sur au moins une partie de leur hauteur s'étendant au-dessous et au-dessus desdits orifices de passage des bras (10, 11), lesquels panneaux intérieur (22a) et extérieur (21a) sont reliés par une zone de jonction (23) conformée pour venir épouser au moins une partie de la hauteur du bord libre (13) de ladite paroi frontale (2), ladite zone de jonction (23) comportant une échancrure (38) conformée pour venir épouser la bordure dudit second orifice de passage (11), et lesquels panneaux intérieur (22a) et extérieur (21a) sont chacun munis d'une ouverture (30, 33) destinée à venir se placer de part et d'autre dudit premier orifice de passage (10), l'ouverture (30, 33) de l'un au moins desdits panneaux (21a, 22a) étant munie d'un élément tubulaire souple (34) formant une sorte de manchette, ladite manchette (34) étant destinée à être passée au travers dudit premier orifice (10), cela de sorte à recouvrir sa bordure et garantir l'asepsie dudit orifice (10).

2. Housse stérile selon la revendication 1, **caractérisée en ce que** les panneaux intérieur (22a) et/ou extérieur (21a) sont prolongés par un ou des panneaux (22b, 22c ; 21b destinés à venir recouvrir les faces intérieure et/ou extérieure de la paroi latérale (3).

3. Housse stérile selon l'une quelconque des revendications 1 ou 2, pour un paravent radioprotecteur (1) dont le premier orifice de passage (10) est prolongé vers l'avant par un manchon de protection (12) réalisé en un matériau radioprotecteur, **caractérisée en ce que** l'ouverture (33) du panneau intérieur (22a) est prolongée par une manchette souple (34) dont la longueur est apte à recouvrir la surface interne dudit manchon de protection (12), et aussi au moins une partie de la longueur de sa surface externe.

4. Housse stérile selon la revendication 3, **caractérisée en ce que** l'extrémité libre (35) de la manchette (34) du panneau intérieur (22a) est munie d'un organe élastique, ce dernier étant agencé pour tendre à réduire son diamètre et étant destiné à former une sorte de bracelet de serrage apte à se resserrer sur la surface extérieure du manchon de protection (12) ou sur un support de manchon (12') associé.

5. Housse stérile selon l'une quelconque des revendications 3 ou 4, **caractérisée en ce que** l'ouverture (30) du panneau extérieur (21a) est également prolongée par une manchette souple (31), cette dernière étant destinée à venir recouvrir au moins une partie de la longueur de la surface externe du manchon de protection (12).

6. Housse stérile selon l'une quelconque des revendications 3 à 5, **caractérisée en ce que** la manchette (34) du panneau intérieur (22a) est recouverte d'un élément amovible (44) en forme de gaine dont l'une des extrémités est fermée, ladite gaine (44) étant destinée à faciliter l'opération de passage de ladite manchette (34) au travers du manchon de protection (12) et étant destinée à être retirée après ladite opération de passage.

7. Housse stérile selon l'une quelconque des revendications 1 à 6, **caractérisée en ce qu'**elle comporte une bande supérieure (27, 28) réalisée en matériau plastique transparent.

8. Housse stérile selon la revendication 7, **caractérisée en ce qu'**elle comporte une partie réalisée en matériau non tissé absorbant de type chirurgical, correspondant à une bande inférieure (25, 26) de ladite housse (20), et/ou à la ou aux manchettes (31, 34) équipant les panneaux intérieur (22a) et/ou extérieur (21a), et/ou à la zone de jonction (23) entre lesdits panneaux (21a, 22a), les bordures attenantes entre le matériau plastique transparent et ledit matériau absorbant étant reliées par couture(s).

9. Housse stérile selon l'une quelconque des revendications 1 à 8, **caractérisée en ce qu'**elle comporte des moyens de solidarisation (40, 41) avec le paravent (1).

10. Housse stérile selon la revendication 9, **caractérisée en ce que** les moyens de solidarisation comprennent des ventouses (40) réparties sur la longueur de sa bordure supérieure.

## Claims

1. Sterile cover intended to cover at least part of the height of a screen (1) made of radioprotective material ensuring protection of an operator against the ionizing radiation emissions of the X-ray type or the like, wherein said screen (1), of the type having an L-shaped horizontal cross-section, is made up of a front wall (2) associated with a side wall (3) extending perpendicularly or substantially perpendicularly from said front wall (2), wherein said front wall (2) is provided with two orifices (10, 11) for the passage of the arms of said operator, a first passage orifice (10) being arranged in the vicinity of the angle formed by said front (2) and side (3) walls, a second passage orifice (11) being located at the free edge (13) of said front wall (2) and being laterally open, thus having the general form of a horizontally oriented circular arc or U-shaped indentation,
**characterized in that** said sterile cover (20) is in the form of a single part, which comprises at least two panels, one being referred to as the "inner panel" (22a) and the other being referred to as the "outer panel" (21a), shaped so as to cover at least the inner and outer faces of said front wall (2), respectively, over at least part of their height extending below and above said arm passage orifices (10, 11), wherein said inner (22a) and outer (21a) panels are connected by a junction region (23) shaped so as to match at least part of the height of the free edge (13) of said front wall (2), said junction region (23) comprising an indentation (38) shaped so as to match the edge of said second passage orifice (11), and wherein said inner (22a) and outer (21a) panels are each provided with an opening (30, 33) intended to be placed on either side of said first passage orifice (10), the opening (30, 33) of at least one of said panels (21a, 22a) being provided with a flexible tubular element (34) forming a kind of cuff, said cuff (34) being intended to be passed through said first orifice (10), so as to cover the edge thereof and to guarantee asepsis of said orifice (10),

2. Sterile cover according to claim 1, **characterized in that** the inner (22a) and/or outer (21a) panels are extended by one or several panels (22b, 22c ; 21b) intended to cover the inner and/or outer faces of the side wall (3).

3. Sterile cover according to any one of claims 1 or 2, for a radioprotective screen (1), the first passage orifice (10) of which is extended forward by a protective sleeve (12) made of radioprotective material, **characterized in that** the opening (33) of the inner panel (22a) is extended by a flexible cuff (34), the length of which is adapted to cover the inner surface of said protective sleeve (12), and also at least part of the length of the outer surface thereof.

4. Sterile cover according to claim 3, **characterized in that** the free end (35) of the cuff (34) of the inner panel (22a) is provided with an elastic member, the latter being arranged so as to tend to reduce the diameter thereof and being intended to form a king of tightening strap adapted to tighten around the outer surface of the protective sleeve (12) or an associated sleeve support (12').

5. Sterile cover according to any one of claims 3 or 4, **characterized in that** the opening (30) of the outer panel (21a) is also extended by a flexible cuff (31), the latter being intended to cover at least part of the length of the outer surface of the protective sleeve (12).

6. Sterile cover according to any one of claims 3 to 5, **characterized in that** the cuff (34) of the inner panel (22a) is covered with a sheath-shaped removable element (44), one end of which is closed, said sheath (44) being intended to facilitate the passage operation of said cuff (34) through the protective sleeve (12) and being intended to be removed after said passage operation.

7. Sterile cover according to any one of claims 1 to 6, **characterized in that** it comprises an upper band (27, 28) made of transparent plastic material.

8. Sterile cover according to claim 7, **characterized in that** it comprises a part made of surgical-type absorbent non-woven material, corresponding to a lower band (25, 26) of said cover (20), and/or to the cuff(s) (31, 34) equipping the inner (22a) and/or outer (21a) panels, and/or to the junction region (23) between said panels (21a, 22a), the adjacent edges between the transparent plastic material and said absorbent material being connected together by one or several seams.

9. Sterile cover according to any one of claims 1 to 8, **characterized in that** it comprises means (40, 41) for attaching it to the screen (1).

10. Sterile cover according to claim 9, **characterized in that** the attaching means comprise suction pads (40) distributed over the length of its upper edge.

## Patentansprüche

1. Sterile Abdeckung, die dazu bestimmt ist, mindestens einen Teil der Höhe eines Schirms (1) aus strahlenschützendem Material abzudecken, die den Schutz eines Bedieners vor Emissionen von ionisierenden Strahlungen des Typs Röntgenstrahlen oder dergleichen gewährleistet, wobei der schirm (1) vom Typ mit L-förmigem horizontalem Querschnitt von einer vorderen Wand (2) gebildet ist, die mit einer Seitenwand (3) verbunden ist, die sich im Winkel oder im Wsesentlichen im Winkel von der vorderen Wand (2) weggehend erstreckt, wobei die vordere Wand (2) mit zwei Öffnungen (10, 11) für die Durchführung der Arme des Bedieners versehen ist, wobei eine erste Durchführungsöffnuyng (10) in der Nähe des Winkels, der von der vorderen Wand (2) und der Seitenwand (3) gebildet ist, vorgesehen ist, eine zweite Durchführungesöffnung (11) am freien Rand (13) der vorderen Wand (2) angeordent und seitlich offen ist, wobei sie eine allgemeine Form einer kreisbogenförmigen Kerbe oder eines horizontal ausgerichteten U aufweist,
**dadurch gekennzeichnet, dass** die sterile Abdeckung (20) in Form eines einzigen Stücks vorhanden ist, das mindestens zwei Platten, eine so genannte "Innenplatte" (22a) und eine so genannte "Außenplatte" (21a), umfasst, die derart ausgeführt sind, dass sie mindestens die Innen- bzw. die Außenseite der vorderen Wand (2) abdecken, und zwar auf mindestens einem Teil ihrer Höhe, die sich unter und über den Öffnungen für die Durchführung der Arme (10, 11) erstreckt, wobei die Innenplatte (22a) und die Außenplatte (21a) durch eine Verbindungszone (23) verbunden sind, die derart ausgeführt ist, dass sie sich zumindest an einen Teil der Höhe des freien Randes (13) der vorderen Wand (2) anlegt, webei die Verbindungszone (23) einen bogenförmigen Ausschnitt (38) umfasst, der derart ausgeführt ist, dass er sich an den Rand der zweiten Durchführungsöffnung (11) anlegt, und wobei die Innenplatte (22a) und die Außenplatte (21a) jeweils mit einer Öffnung (30, 33) versehen sind, die dazu bestimmt ist, beiderseits der ersten Durchführungsöffnung (10) angeordnet zu werden, wobei die Öffnung (30, 33) mindestens einer der Platten (21a, 22a) mit einem bigsamen röhrenförmigen Element (34) versehen ist, das eine Art Hülse bildet, wobei die Hülse (34) dazu bestimmt ist über die erste Öffnung (10) geschoben zu werden, um ihren Rand abzudecken und die Keimfreiheit der Öffnung (10) zu gewährleisten.

2. Sterile Abdeckung nach Anspruch 1, **dadurch gekennzeichnet, dass** die Innenplatte (22a) und/oder die Außenplatte (21a) durch eine oder mehrere platten (22b, 22c; 21b) verlängert sind, die dazu bestimmt sind, die Inner- und/oder Außenseite der Seitenwand (3) abzudecken.

3. Sterile Abdeckung nach einem der Ansprüche 1 oder 2 für einen Strahlenschutzschirm (1), deren erste Durchführungsöffnung (10) nach vorne durch eine Schutzhülse (12), die aus einem strahlenschützenden Material hergestellt ist, verlängert ist, **dadurch gekennzeichnet, dass** die Öffnung (33) der Innenplatte (22a) durch eine biegsame Hülse (34) verlängert ist, deren Länge geeigenet ist, die Innenfläche der Schutzhülse (12) und zumindest einen Teil der Länge ihrer Außenfläche abzudecken.

4. Sterile Abdeckung nach Anspruch 3, **dadurch gekennzeichnet, dass** das freie Ende (35) der Hülse (34) der Innenplatte (22a) mit einem elastischen Element versehen ist, wobei dieses letztgenannte derart vorgesehen ist, dass es dazu neigt, seinen Durchmesser zu verringern, und dazu bestimmt ist, eine Art Spannband zu bilden, das geeignet ist, sich auf der Außenfläche der Schutzhülse (12) oder auf einer zugehörigen Hülsenstütze (12') zu spannen.

5. Sterile Abdeckung nach einem der Ansprüche 3 oder 4, **dadurch gekennzeichnet, dass** die Öffnung (30) der Außenplatte (21a) ebenfalls durch eine biegsame Hülse (31) verlängert ist, wobei diese letztgenannte dazu bestimmt ist, zumindest einen Teil der Länge der Außenfläche der Schutzhülse (12) abzudecken.

6. Sterile Abdeckung nach einem der Ansprüche 3 bis 5, **dadurch gekennzeichnet, dass** die Hülse (34) der Innenplatte (22a) mit einem abnehmbaren Element (44) in Form eines Mantels abgedeckt ist, wobei eines seiner Enden verschlossen ist, wobei der Mantel (44) dazu bestimmt ist, die Durchführung der Hülse (34) durch die Schutzmanschette (12) zu erleichtern und nach der Durchführung herausgezogen zu werden.

7. Sterile Abdeckung nach einem der Ansprüche 1 bis 6, **dadurch gekennzeichnet, dass** sie ein oberes Band (27, 28) umfasst, das aus einem transparenten kunststoff hergestellt ist.

8. Sterile Abdeckung nach Anspruch 7, **dadurch gekennzeichenet, dass** sie einen Teil umfasst, der aus einem absorbierenden Faservlies für Chirurgen hergestellt ist, entsprechend einem unteren Band (25, 26) der Abdeckung (20) und/oder der oder den Hülsen (31, 34), mit denen die Innenplatte (22a) und/oder die Außenplatte (21a) versehen sind, und/oder der Verbindungszone (23) zwischen den Platten (21a, 22a), wobei die angrenzenden Ränder zwischen dem transparentent Kunststoff und dem absorbierenden Material durch Nähte verbunden sind.

9. Sterile Abdeckung nach einem der Ansprüche 1 bis 8, **dadurch genkennzeichnet, dass** sie Mittel (40, 41) zur Verbindung mit dem Schirm (1) umfasst.

10. Sterile Abdeckung nach Anspruch 9, **dadurch gekennzeichnet, dass** die Verbindungsmittel Saugnäpfe (40) umfassen, die auf der Länge ihres oberen Randes verteilt sind.
